# EUROPEAN PATENT APPLICATION

(11) **EP 4 027 656 A1**
(43) Date of publication of application: **13.07.2022**
(21) Application number: 21150708.2
(22) Date of filing: 08.01.2021
(51) Int. Cl.: H04R 25/00, A61B 5/01

(54) **HEARING AID FOR OBTAINING A TEMPERATURE, AND A METHOD THEREOF**

(71) Applicant: Oticon A/S, 2765 Smørum (DK)
(72) Inventor: KVIST, Preben, 2765 Smørum (DK)
(74) Representative: Nielsen, Hans Jørgen Vind

(57) **Abstract**

The present application relates to a hearing aid having a first housing (1) configured to be positioned at least partly in the ear canal (EC) of a user. The first housing (1) comprises a receiver (2) with a receiver outlet (4), a flexible dome (3) configured on the receiver (2) such that a residual space (RS) is formed between the dome (3) and the inner part of the user's ear canal (EC). The flexible dome (3) comprises a sensor outlet channel (6), separate from the receiver outlet channel (5), in which a first temperature sensor (7) is arranged for obtaining a temperature in the residual space (RS).

## Description

### SUMMARY

The present application relates to a hearing aid comprising a temperature sensor. The present application further relates to a method for estimating a user's body temperature.

Hearing aids may be provided in different configurations, such as commonly denoted In-the-Ear, Receiver-In-the-Ear, Behind-the-Ear and so forth.

In-the-ear hearing aid devices (ITE) usually comprise an exterior housing (of an in-the-ear unit thereof) and an electro-acoustic output transducer arranged therein. Receiver-In-the-Ear (RITE) hearing aid devices usually comprise an exterior housing configured to be positioned behind the ear of the user, with an electro-acoustic output transducer arranged in a separate housing configured to be placed at least partly in the ear canal of the user. Behind-the-Ear hearing aid devices (BTE) usually comprise an exterior housing configured to be positioned behind the ear of the user, with an electro-acoustic output transducer arranged in a the same housing and be configured to be connected to a hollow tube arranged for guiding acoustic sound from the output transducer to the ear canal of the user.

The electro-acoustic output transducer is often termed a receiver and is configured to convert an electric audio signal into an acoustic sound signal. The electric audio signal may be provided by a sound processor. The sound processor may receive an electric audio input signal and process the electric audio input signal to generate a processed electric audio signal to be fed to the output transducer. The sound processor may process the sound so as to compensate for a specific hearing loss of the intended user.

The sound processor may be provided in a behind-the-ear unit of the hearing aid or for the ITE hearing aid in the housing configured to be positioned in or at the ear canal. The electric audio input signal may be received from an electro-acoustic input transducer. Alternatively, or in combination herewith, the electric audio input signal may be received wirelessly, e.g. via an RF radio channel, such as via a Bluetooth connection or the like, or other types of wireless signals, such as telecoil, inductive systems or the like. The electro-acoustic input transducer may be provided in the in-the-ear unit or may be provided in the BTE unit. The electro-acoustic input transducer is called microphone and converts an acoustic sound signal into an electric audio signal.

In RITE hearing aids, the sound processor is typically arranged in the BTE unit and is connected to the electro-acoustic output transducer (and the electro-acoustic input transducer) by way of electrically conducting wires arranged in a coupling element (connection tube) that mechanically connects the in-the-ear unit to the behind-the-ear unit.

When people wear a hearing aid, the device is usually placed in close contact with the head of the user, and if a temperature sensor is included and arranged as close to the ear drum as possible, within the hearing aid housing or the in-the-ear-canal housing having the receiver as well, it is possible to obtain a measurement of the temperature in the ear canal, which may be used as an estimate of the core temperature of the user (herein also referred to as "the body temperature"). This, first, temperature sensor is thus utilized to establish an estimate of the core temperature of the person wearing the hearing instrument from inside the ear(canal) using a sensor attached to the receiver. In addition to the usual measurement insecurities, other factors may degrade the estimate.

Therefore, there is a need to provide a solution that at least provides an improved estimate of the body temperature of the person wearing the hearing aid, and at least alleviates some of the above-mentioned problems. The present disclosure provides at least an alternative to the prior art.

### A hearing aid:

In an aspect of the present invention, a hearing aid having a first housing, the first housing being configured to be positioned at least partly in the ear canal of a user, the first housing comprising a receiver having a receiver outlet, a flexible dome configured on the receiver, such that a residual space is formed between the dome and the inner part of the user's ear canal, and a receiver outlet channel in the dome for providing a fluid connection between the receiver outlet and the residual space, **Error! Reference source not found.**is provided. The flexible dome may further comprise a sensor outlet channel, separate from the receiver outlet channel, in which a first temperature sensor is arranged for obtaining a temperature in the residual space. Thereby, an improved estimate of the body temperature can be facilitated, since the temperature of the ear drum, and thereby the residual space, is closely related to the core body temperature. A further advantage of placing a temperature sensor in the dome is that it doesn't take up unnecessary space. Another advantage is that it doesn't have to be in direct contact with the skin of the user, thus alleviating discomfort.

The hearing aid may further comprise a temperature processor configured to process signals from the first temperature sensor.

The hearing aid may further comprise a second temperature sensor in communication with the temperature processor, wherein the temperature processor estimates the user's body temperature based on the measurements from both the first and second temperature sensors. An advantage of utilizing multiple temperature sensors is that the estimate of the user's body temperature becomes more accurate.

The hearing aid may further comprise a second housing, wherein the second temperature sensor is placed in the second housing.

The second housing may be configured to be positioned behind the ear of the user, and/or in the concha of the user, and/or in the fossa triangularis. An advantage of this is that positioning the housing outside of the inner ear canal allows for measuring ambient temperatures, which facilitates a more accurate estimate of the user's body temperature.

The hearing aid may comprise a second temperature sensor placed in an external device in wired or wireless communication with the temperature processor.

The hearing aid may further comprise a memory for storing measured temperature values. An advantage of this is that sensor data (i.e. measured temperature values) can be logged and tracked over a period of time, thus accounting for the fluctuations in body temperature throughout the day.

The hearing aid may further comprise a microphone arranged in connection to the sensor outlet channel, and the first temperature sensor and the microphone may form a single unit. An advantage of this is that a microphone facing into the ear canal can be used for estimating the sound pressure at the ear drum of the user, thus aiding in alleviating problems such as occlusion. By arranging the temperature sensor and the microphone together in a single unit, valuable space in the hearing aid can be saved.

The hearing aid may further comprise a microphone arranged in connection to the receiver outlet channel. An advantage of this is that a microphone facing into the ear canal can be used for estimating the sound pressure at the ear drum of the user, thus aiding in alleviating problems such as occlusion.

The hearing aid may comprise a flexible dome being a silicone dome. An advantage of this is that silicone has low heat conduction properties which is beneficial when measuring temperatures. Other possible materials are 2K epoxies. It is further possible that the dome is coated to better combat for example human sweat and body heat.

### A method:

In a second aspect of the present invention, a method for estimating a user's body temperature is provided. The method comprises placing a hearing aid having a first housing at least partly in the ear canal, wherein the first housing comprises a receiver having a receiver outlet, a flexible dome, configured on the receiver, such that a residual space is formed between the dome and the inner part of the user's ear canal, and a receiver outlet channel in the dome for providing a fluid connection between the receiver outlet and the residual space, wherein the flexible dome comprises a sensor outlet channel, separate from the receiver outlet channel, in which a first temperature sensor is arranged, and obtaining data from the first temperature sensor in the residual space. The user's body temperature may be estimated based on the data obtained from the first temperature sensor.
Thereby, an improved estimate of the body temperature can be facilitated.

The method may further comprise a second temperature sensor, and further comprise the steps of: obtaining data from the second temperature sensor, and estimating the user's body temperature, based on the data from both the first and second temperature sensors.
An advantage of this is that the estimated body temperature will be more accurate.

The method may further comprise that the second temperature sensor is arranged in an external device in wired or wireless communication with the temperature processor, and further comprise the step of communicating the second temperature sensor data from the external device to the temperature processor.

It is intended that some or all of the structural features of the hearing aid described above, in the 'detailed description of embodiments' or in the claims can be combined with embodiments of the method for estimating a user's body temperature, when appropriately substituted by a corresponding process and vice versa. Embodiments of the method for estimating a user's body temperature have the same advantages as the corresponding hearing aids.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
FIG. 1 shows a schematic view of a hearing aid in an ear,
FIG. 2 shows a hearing aid according to the invention,
FIG. 3 shows a hearing aid according to the invention,
FIG. 4 shows the steps of a method according to the invention,
FIG. 5 shows the steps of a method according to the invention,
FIG. 6 shows the steps of a method according to the invention.

The figures are schematic and simplified for clarity, and they just show details which are essential to the understanding of the disclosure, while other details are left out. Throughout, the same reference signs are used for identical or corresponding parts.

Further scope of applicability of the present disclosure will become apparent from the detailed description given hereinafter. However, it should be understood that the detailed description and specific examples, while indicating preferred embodiments of the disclosure, are given by way of illustration only. Other embodiments may become apparent to those skilled in the art from the following detailed description.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

In the present context, a hearing aid, e.g. a hearing instrument, refers to a device, which is adapted to improve, augment and/or protect the hearing capability of a user by receiving acoustic signals from the user's surroundings, generating corresponding audio signals, possibly modifying the audio signals and providing the possibly modified audio signals as audible signals to at least one of the user's ears. Such audible signals may e.g. be provided in the form of acoustic signals radiated into the user's outer ears, acoustic signals transferred as mechanical vibrations to the user's inner ears through the bone structure of the user's head and/or through parts of the middle ear as well as electric signals transferred directly or indirectly to the cochlear nerve and/or auditory cortex of the user.

The hearing aid may be configured to be worn in any known way, e.g. as a unit arranged behind the ear with a tube leading radiated acoustic signals into the ear canal or with an output transducer, e.g. a loudspeaker, arranged close to or in the ear canal, as a unit entirely or partly arranged in the pinna and/or in the ear canal, as a unit, e.g. a vibrator, attached to a fixture implanted into the skull bone, as an attachable, or entirely or partly implanted, unit, etc. The hearing aid may comprise a single unit or several units communicating (e.g. acoustically, electrically or optically) with each other. The loudspeaker may be arranged in a housing together with other components of the hearing aid, or may be an external unit in itself (possibly in combination with a flexible guiding element, e.g. a dome-like element).

More generally, a hearing aid comprises an input transducer for receiving an acoustic signal from a user's surroundings and providing a corresponding input audio signal and/or a receiver for electronically (i.e. wired or wirelessly) receiving an input audio signal, a (typically configurable) signal processing circuit (e.g. a signal processor, e.g. comprising a configurable (programmable) processor, e.g. a digital signal processor) for processing the input audio signal and an output unit for providing an audible signal to the user in dependence on the processed audio signal. The signal processor may be adapted to process the input signal in the time domain or in a number of frequency bands. In some hearing aids, an amplifier and/or compressor may constitute the signal processing circuit. The signal processing circuit typically comprises one or more (integrated or separate) memory elements for executing programs and/or for storing parameters used (or potentially used) in the processing and/or for storing information relevant for the function of the hearing aid and/or for storing information (e.g. processed information, e.g. provided by the signal processing circuit), e.g. for use in connection with an interface to a user and/or an interface to a programming device. In some hearing aids, the output unit may comprise an output transducer, such as e.g. a loudspeaker for providing an air-borne acoustic signal or a vibrator for providing a structure-borne or liquid-borne acoustic signal. In some hearing aids, the output unit may comprise one or more output electrodes for providing electric signals (e.g. to a multi-electrode array) for electrically stimulating the cochlear nerve (cochlear implant type hearing aid).

In some hearing aids, the vibrator may be adapted to provide a structure-borne acoustic signal transcutaneously or percutaneously to the skull bone. In some hearing aids, the vibrator may be implanted in the middle ear and/or in the inner ear. In some hearing aids, the vibrator may be adapted to provide a structure-borne acoustic signal to a middle-ear bone and/or to the cochlea. In some hearing aids, the vibrator may be adapted to provide a liquid-borne acoustic signal to the cochlear liquid, e.g. through the oval window. In some hearing aids, the output electrodes may be implanted in the cochlea or on the inside of the skull bone and may be adapted to provide the electric signals to the hair cells of the cochlea, to one or more hearing nerves, to the auditory brainstem, to the auditory midbrain, to the auditory cortex and/or to other parts of the cerebral cortex.

A hearing aid may be adapted to a particular user's needs, e.g. a hearing impairment. A configurable signal processing circuit of the hearing aid may be adapted to apply a frequency and level dependent compressive amplification of an input signal. A customized frequency and level dependent gain (amplification or compression) may be determined in a fitting process by a fitting system based on a user's hearing data, e.g. an audiogram, using a fitting rationale (e.g. adapted to speech). The frequency and level dependent gain may e.g. be embodied in processing parameters, e.g. uploaded to the hearing aid via an interface to a programming device (fitting system), and used by a processing algorithm executed by the configurable signal processing circuit of the hearing aid.

The electronic hardware may include micro-electronic-mechanical systems (MEMS), integrated circuits (e.g. application specific), microprocessors, microcontrollers, digital signal processors (DSPs), field programmable gate arrays (FPGAs), programmable logic devices (PLDs), gated logic, discrete hardware circuits, printed circuit boards (PCB) (e.g. flexible PCBs), and other suitable hardware configured to perform the various functionality described throughout this disclosure, e.g. sensors, e.g. for sensing and/or registering physical properties of the environment, the device, the user, etc. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

A "hearing system" refers to a system comprising one or two hearing aid devices, and a "binaural hearing system" refers to a system comprising two hearing aid devices where the devices are adapted to cooperatively provide audible signals to both of the user's ears. The hearing system or binaural hearing system may further include auxiliary device(s) that communicates with at least one hearing aid device, the auxiliary device affecting the operation of the hearing aid devices and/or benefitting from the functioning of the hearing aid devices. A wired or wireless communication link between the at least one hearing aid device and the auxiliary device is established that allows for exchanging information (e.g. control and status signals, possibly audio signals) between the at least one hearing aid device and the auxiliary device. Such auxiliary devices may include at least one of remote controls, remote microphones, audio gateway devices, mobile phones, public-address systems, car audio systems or music players or a combination thereof. The audio gateway is adapted to receive a multitude of audio signals such as from an entertainment device like a TV or a music player, a telephone apparatus like a mobile telephone or a computer, a PC. The audio gateway is further adapted to select and/or combine an appropriate one of the received audio signals (or combination of signals) for transmission to the at least one hearing aid device. The remote control is adapted to control functionality and operation of the at least one hearing aid devices. The function of the remote control may be implemented in a SmartPhone or other electronic device, the SmartPhone/ electronic device possibly running an application that controls functionality of the at least one hearing aid device.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method is not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein but are to be accorded the full scope consistent with the language of the claims, wherein reference to an element in the singular is not intended to mean "one and only one" unless specifically so stated, but rather "one or more." Unless specifically stated otherwise, the term "some" refers to one or more.

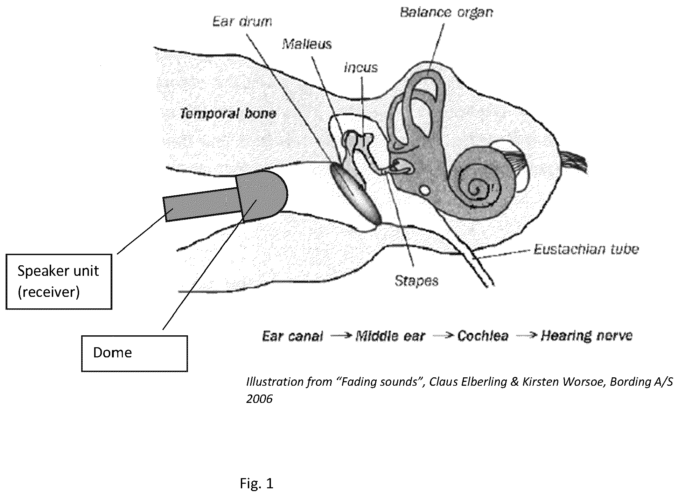

Figure 1 illustrates the placement of a hearing aid in a human ear, with the so called "in-the-ear" part of the hearing aid arranged in the ear canal. The in-the-ear part in figure 1 comprises a speaker unit, and a dome in close proximity to the ear drum. Although not shown in figure 1, the in-the-ear part can be connected to a "behind-the-ear" part of the hearing aid, placed behind the ear outside of the user's ear canal.

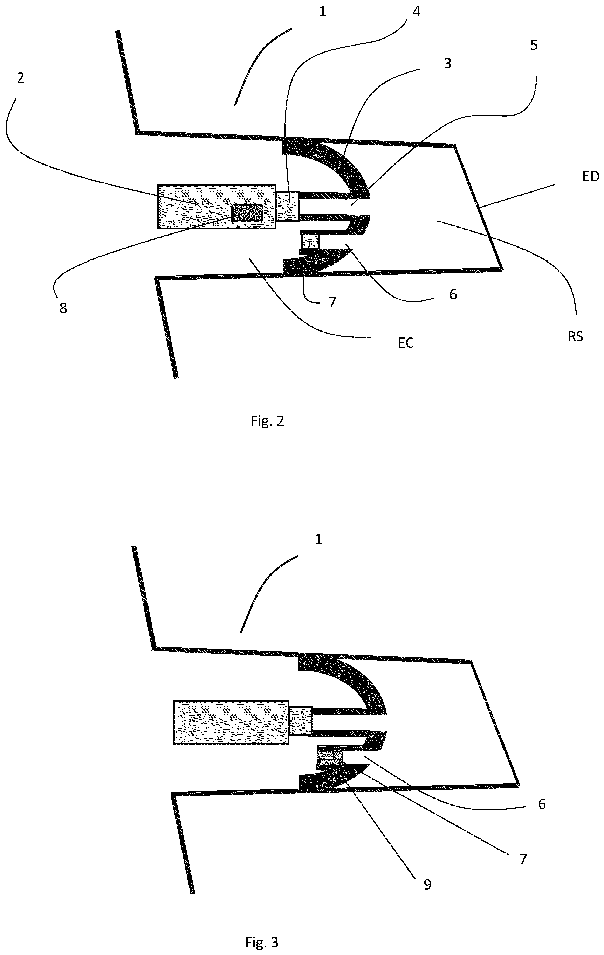

Figure 2 shows a hearing aid according to the present invention, placed in an ear in a similar manner as in figure 1. The hearing aid comprises a first housing 1, positioned at least partly in the ear canal EC of the user. The first housing 1 comprises a receiver 2, and a dome 3. As can be seen in figure 2, the dome 3 is mounted on the receiver 2 and is placed in proximity to the user's ear drum ED. The dome can be flexible, such as made of silicone or any other suitable material. Just like in figure 1, the hearing aid may comprise other parts, such as housings and/or wires connected to the first housing 1. The receiver 2 in figure 2 is shown located fully in the ear canal EC, it is however possible that parts of the receiver 2 is located outside of the ear canal EC. The receiver 2 has a receiver outlet 4 for letting sound out. As can be seen, a residual space RS is formed between the dome 3 and the user's ear drum ED in the inner part of the ear canal EC. The receiver outlet 4 is in fluid connection with this residual space RS through a passage 5 in the dome 3, which forms a receiver outlet channel 5. In addition to the receiver outlet channel 5, the dome 3 comprises another outlet channel 6, separate from the receiver outlet channel 5. A sensor 7 is placed in the outlet channel 6, hereinafter called the sensor outlet channel 6. The sensor 7 is a sensor suitable for measuring biological parameters, such as a temperature sensor. Since the temperature of the ear drum ED closely corresponds to the core body temperature of the user, it is very beneficial to measure the temperature close to the ear drum ED, i.e. in the residual space RS. One example of a suitable temperature sensor 7 is an infrared thermometer. An infrared thermometer doesn't require direct contact with the ear drum ED, which is advantageous since constant contact would cause discomfort for the user. It is further possible that other biological parameters relating to physiological parameters of the patient are measured with the sensor 7, such as voltage, current, pressure, flow rate, blood pressure, blood saturation etc. In figure 2, one sensor outlet channel 6 and one temperature sensor 7 is shown, it is however plausible that the dome 3 comprises multiple sensor outlet channels 6 and/or multiple sensors 7. In figure 2, the first housing is shown comprising a dome 3, it is however obvious to someone skilled in the art, that other hearing aid styles with different shapes can be used, such as ITE-styles without exterior housings.
The hearing aid can further comprise a temperature processor, not shown, for processing data from the temperature sensor 7. The temperature processor can be located in a second housing, such as a behind-the-ear part of the hearing aid, not shown. The temperature processor is configured to estimate the body temperature of the user, based on the measured data from the sensor 7. Although the hearing aid shown in figure 2 shows only one first sensor 7, it can be advantageous to have a second temperature sensor, not shown. The second temperature sensor can be in wired or wireless communication with the temperature processor. The temperature processor can utilize measured data from both the first 7 and second sensors when estimating the body temperature. It is possible that the second temperature sensor is located in or on a second housing of the hearing aid. The second housing can, as mentioned, be placed behind the ear of the user, and/or the in the concha, and/or in the fossa triangularis. The second temperature sensor, if placed outside of the ear canal EC, can be used as a reference sensor. The temperature data from the reference sensor, is used to correct for variations in ambient temperature, as there will be a thermal energy conduction through wires connecting the in-ear sensor (first sensor 7) and the temperature processor positioned behind the ear, i.e. behind the pinna, and radiated heat in and out of the ear canal, which all will influence measurements. Using the second temperature sensor as a reference will also be possible for ITE-style hearing aids without exterior housings, where the 'ambient temperature' is measured with a reference sensor placed on the faceplate of ,for example, the custom instrument, and thus not as such external to the ear, but at least in a distance from the first temperature sensor 7. Information, or data, about ambient temperature can also be gathered from external devices instead of using a reference sensor positioned behind/outside the ear of the user. This could include external auxiliary devices, such as a microphone device configured to be positioned at a table and/or on another person whom the user intends to hear better. Also, temperature may be obtained from internet enabled devices, e.g. thermometers with IoT capabilities. The external auxiliary device can be in wired or wireless communication with the temperature processor.
The hearing aid can further comprise a memory 8. As can be seen in figure 2, the memory can be located in the in-the-ear part of the hearing aid. The memory 8 can store measured data from the sensor 7. The sensor 7 is connected to the memory 8 wired or wirelessly. The memory 8 allows measured sensor 7 data to be logged for a period time, which can be especially useful when trying to estimate the body temperature. The body temperature fluctuates during the day, and is affected by activity, illness etc. One way of using the measured data over time is to use averaging and /or time stamping methods to calculate the body temperature. By logging data over time, useful statistics can also be gathered that is connected to the user's body temperature pattern.
In figure 3, a hearing aid according to the present invention is shown. The first housing 1 in figure 3 is similar to the one shown in figure 2, with the exception of an added microphone 9. The microphone 9 is placed in connection with the sensor 7 in the sensor outlet channel 6. The sensor 7 and the microphone 9 can be formed in a single unit. Although in figure 3, the microphone 9 is shown as being placed in the sensor outlet 6, it is also possible that it is placed in connection to the receiver outlet channel 5.

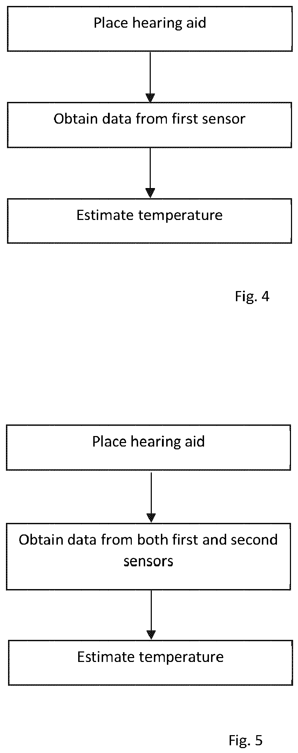

Figure 4 shows the steps of a method for estimating a user's body temperature according to the invention. The first step is to place the hearing aid with a first housing at least partly in the ear canal of the user. The hearing aid can be a hearing aid as described above with reference to figures 2 and/or 3. The second step of the method is to obtain data from the first temperature sensor 7. The temperature sensor 7 is placed near the user's ear drum ED in the residual space RS and the obtained data therefor corresponds to the user's core body temperature. The user's body temperature is thus estimated in the last step as the temperature obtained by the sensor 7. The estimation is performed in the temperature processor located in the hearing aid, and to which the temperature sensor is connected wired or wirelessly.

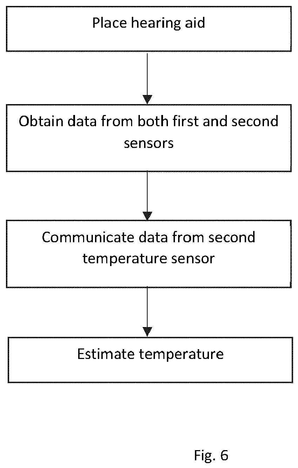

Figure 5 shows the steps of a second method for estimating a user's body temperature according to the invention. As opposed to the method described in figure 4, this method comprises a hearing aid also having a second sensor, further to the first sensor 7. Thus, the step of obtaining data includes obtaining data from both the first 7 and second sensors. The second sensor is preferably located outside the user's ear canal EC. The second sensor is possibly located in an external device, in wired or wireless connection with the temperature processor, as figure 6 shows. The method shown in figure 6 thus comprises the step of communicating the data from the second sensor to the temperature processor located in the hearing aid. The external device may for example be a Smartphone. A communication part of the hearing aid which is configured for communication with an external device may be provided, for example, in the first housing 1 or in the behind-the-ear part of the hearing aid and may comprise, for example, a transmission and/or reception controlling circuitry and an antenna. While it is preferable to provide the communication part in the behind-the-ear part of the hearing aid for keeping the in-the-ear part 1 of the hearing aid as small as possible, it is also possible to integrate the communication in the in-the-ear part 1 of the hearing aid. At least, the second sensor is connected to the communication part. By means of the communication part, the hearing aid may be connected with the external device e.g. via wireless local area network (WLAN), Bluetooth low energy, Nearlink or other techniques and can share the measured data wirelessly to the hearing aid. The connection between the second sensor and/or the active electronic component in the hearing aid may be initiated by the second sensor and/or the active electronic component or by the external device. The connection may be established by a signal processor arranged within the hearing aid based on a security signal provided by a security mean. The security mean may receive a request signal from the external device, wherein the request signal includes an identification code identifying the external device. The security mean may accept the request signal if the identification (ID) code is identical to a stored ID code in a volatile memory/none-volatile memory being arranged within the hearing aid. When the measured data from both the second and first sensors are obtained and communicated to the temperature processor, the user's body temperature can be estimated. The second sensor may measure ambient temperatures and may benefit from being as far away from the first sensor 7 as possible as such measurement would be less influenced by the body heat of the user.

It is a further possibility that the temperature processor is located in an external device, in order to save precious space and energy in the hearing aid. In such a case, the sensor(s) can communicate wirelessly to the external device.

The hearing aid and method according to the present disclosure could utilize additional sensor inputs, such as heart rate (variability), oxygenation, accelerometer, microphones or ambient light sensing to improve reliability by providing more inputs to the algorithm. E.g. detecting physical activity, movement, wind or sunlight.

### REFERENCES

- First housing 1
- Receiver 2
- Dome 3
- Receiver outlet 4
- Receiver outlet channel 5
- Sensor outlet channel 6
- First sensor 7
- Memory 8
- Ear canal EC
- Ear drum ED
- Residual space RS

## Claims

1. A hearing aid having a first housing (1), the first housing (1) being configured to be positioned at least partly in the ear canal (EC) of a user, the first housing (1) comprising:
a receiver (2) having a receiver outlet (4),
a flexible dome (3), configured on the receiver (2), such that a residual space (RS) is formed between the flexible dome (3) and the inner part of the user's ear canal (EC),
a receiver outlet channel (5) in the flexible dome (3) for providing a fluid connection between the receiver outlet (4) and the residual space (RS),
wherein the flexible dome (3) comprises a sensor outlet channel (6), separate from the receiver outlet channel (5), in which a first temperature sensor (7) is arranged for obtaining a measurement representing the temperature in the residual space (RS).

2. Hearing aid according to claim 1, further comprising a temperature processor configured to process measurements from the first temperature sensor.

3. Hearing aid according to claim 2, further comprising a second temperature sensor in communication with the temperature processor, wherein the temperature processor estimates the user's body temperature based on the measurements from both the first and second temperature sensors.

4. Hearing aid according to claim 3, further comprising a second housing, wherein the second temperature sensor is placed in the second housing.

5. Hearing aid according to claim 4, wherein the second housing is configured to be positioned behind the ear of the user, and/or in the concha of the user, and/or in the fossa triangularis.

6. Hearing aid according to claim 3, wherein the second temperature sensor is placed in an external device in wired or wireless communication with the temperature processor.

7. Hearing aid according to any of the preceding claims, further comprising a memory for storing obtained measurements.

8. Hearing aid according to any of the preceding claims, further comprising a microphone arranged in connection with the sensor outlet channel, and wherein the first temperature sensor and the microphone forms a single unit.

9. Hearing aid according to any of the previous claims, further comprising a microphone arranged in connection to the receiver outlet channel.

10. Hearing aid according to any of the previous claims, wherein the flexible dome is a silicone dome.

11. A method for estimating a user's body temperature, the method comprising:
placing a hearing aid having a first housing at least partly in the ear canal, wherein the first housing comprises:
a receiver (2) having a receiver outlet (4),
a flexible dome (3), configured on the receiver (2), such that a residual space (RS) is formed between the flexible dome (3) and the inner part of the user's ear canal (EC),
a receiver outlet channel (5) in the flexible dome (3) for providing a fluid connection between the receiver outlet (4) and the residual space (RS),
wherein the flexible dome (3) comprises a sensor outlet channel (6), separate from the receiver outlet channel (5), in which a first temperature sensor (7) is arranged for obtaining a measurement representing the temperature in the residual space (RS), and
obtaining data from the first temperature sensor in the residual space, and
estimating the user's body temperature based on the data from the first temperature sensor.

12. Method according to claim 11, wherein the hearing aid further comprises a second temperature sensor, the method further comprising the steps of:
obtaining a measurement from the second temperature sensor, and
estimating the user's body temperature, based on the measurements from both the first and second temperature sensors.

13. Method according to claim 12, wherein the second temperature sensor is arranged in an external device in wired or wireless communication with the temperature processor, the method further comprising the step of:
communicating the obtained measurement from the second temperature sensor from the external device to the temperature processor.
